# FASCICULE DE BREVET EUROPEEN

(11) **EP 1 479 366 B1**
(45) Date de publication et mention de la délivrance du brevet: **18.07.2007**
(21) Numéro de dépôt: 04291244.4
(22) Date de dépôt: 14.05.2004
(51) Int. Cl.: A61K 8/81, A61K 8/06, A61K 8/31, A61K 8/894, A61Q 1/02

(54) **Composition de maquillage pour la peau et plus particulièrement une composition de type fond de teint fluide, dotée de qualités d'application optimisées**
Makeup Zusammensetzung bzw. Grundierung mit optimierten Anwendungseigenschaften
Makeup composition and more specifically a fluid foundation with optimised applying properties

(30) Priorité: 22.05.2003 FR 0306150
(43) Date de publication de la demande: 24.11.2004
(73) Titulaire: L'ORÉAL, 75008 Paris (FR)
(72) Inventeur: Gardel, Nadia, 92340 Bourg la Reine (FR); Barrois, Véronique, 75011 Paris (FR)
(74) Mandataire: Le Coupanec, Pascale A.M.P.

(56) Documents cités:
- US-A- 5 340 582
- US-B1- 6 254 877
- US-B1- 6 326 013
- PATENT ABSTRACTS OF JAPAN vol. 2003, no. 01, 14 janvier 2003 (2003-01-14) & JP 2002 265333 A (SHISEIDO CO LTD), 18 septembre 2002 (2002-09-18)
- PATENT ABSTRACTS OF JAPAN vol. 2002, no. 05, 3 mai 2002 (2002-05-03) & JP 2002 020234 A (POLA CHEM IND INC), 23 janvier 2002 (2002-01-23)
- PATENT ABSTRACTS OF JAPAN vol. 014, no. 242 (C-0721), 23 mai 1990 (1990-05-23) & JP 02 062816 A (NIPPON SHIKIZAI KOGYO KENKYUSHO:KK), 2 mars 1990 (1990-03-02)
- PATENT ABSTRACTS OF JAPAN vol. 2003, no. 01, 14 janvier 2003 (2003-01-14) & JP 2002 265620 A (SEKISUI PLASTICS CO LTD), 18 septembre 2002 (2002-09-18)

## Description

La présente invention concerne une composition de maquillage pour la peau et plus particulièrement une composition de type fond de teint fluide, dotée de qualités d'application optimisées.

D'une manière générale, l'obtention d'un maquillage satisfaisant de la peau à l'aide d'un fond de teint implique de pouvoir répartir celui-ci de manière homogène sur l'ensemble de la surface à maquiller. Pour ce faire, il est nécessaire que la formulation de fond de teint possède des qualités de glissant suffisantes pour se prêter à une bonne maniabilité, notamment en vue d'assurer la couvrance de toute la surface à maquiller. C'est ce qui permet notamment d'obtenir au final un maquillage homogène, sans trace ni démarcation.

Plus particulièrement, les inventeurs ont mis en évidence que la présence d'une quantité efficace de polyméthacrylate de méthyle dit encore PMMA dans certaines compositions de type fonds de teint était précisément avantageuse en ces termes.

Les polyméthacrylates de méthyle se présentent généralement sous la forme de particules sphériques creuses de couleur blanche dont la taille est généralement à l'échelle du micromètre. Ces particules ont pour particularité de présenter une forte capacité d'àdsorption d'eau ou d'huile particulièrement avantageuse dans les formulations cosmétiques. Ainsi, dans des compositions cosmétiques à base d'huiles, en adsorbant en partie ces huiles, elles permettent de conférer à la composition correspondante un toucher sec et poudreux. Par ailleurs, dans les formulations de type base rouge à lèvres, elles permettent d'incorporer 10 à 30 % d'eau. Ces particules sphériques creuses de polyméthacrylate de méthyle ont ainsi déjà été proposées à des fins d'encapsulation ou de réservoir, dans des huiles sèches, des rouges à lèvres aqueux, des poudres libres ou compactes à teneur élevée en huile ou en eau.

De manière inattendue, les inventeurs ont constaté que la présence de polyméthacrylate de méthyle pouvait être également déterminante pour optimiser les propriétés des compositions cosmétiques à l'image notamment des fonds de teint dits fluides, en terme d'application.

En conséquence, la présente invention a pour objet principal selon un premier aspect, une composition cosmétique fluide sous forme d'une émulsion eau-dans-huile comprenant une phase grasse liquide, une phase aqueuse et un diméthiconecopolyol, caractérisée en ce qu'elle comprend des particules sphériques solides de polyméthacrylate de méthyle et que la phase grasse liquide comprend de l'isododécane, ladite composition étant exempte de cyclotétrasiloxane.

La présente invention vise également selon un second aspect, une composition cosmétique fluide sous forme d'une émulsion eau-dans-huile comprenant une phase grasse liquide, une phase aqueuse, un diméthiconecopolyol et un alkyl C₈-C₂₂ diméthicone copolyol, caractérisée en ce qu'elle comprend des particules sphériques solides de polyméthacrylate de méthyle et que la phase grasse liquide comprend une huile hydrocarbonée volatile, ladite composition étant exempte de cyclotétrasiloxane.

La présente invention mise également selon un troisième aspect, une composition cosmétique fluide sous forme d'une émulsion eau-dans-huile comprenant une phase grasse liquide, une phase aqueuse et un diméthicone copolyol, caractérisée en ce qu'elle comprend des particules solides de polyméthacrylate de méthyle avec au moins deux polyméthacrylates de méthyle différents en terme de densité et en ce que la phase grasse liquide comprend de l'isododécane, ladite composition étant exempte de cyclotétrasiloxane.

La présente invention a également pour objet selon un autre de ses aspects, un procédé de maquillage de la peau, caractérisé en ce qu'il comprend l'application sur la peau d'au moins une composition conforme à l'invention.

### Polyméthacrylates de méthyle

Les polyméthacrylates de méthyle se présentent généralement sous la forme de particules sphériques creuses ou pleines de couleur blanche dont la taille moyenne en nombre est généralement à l'échelle du micromètre, en particulier varie de 5 à 20 microns et généralement varie de 7 à 15 microns. Par « taille moyenne en nombre », on désigne la dimension donnée par la distribution granulométrique statistique à la moitié de la population, dite D50.

Il est également possible de caractériser ces particules de polyméthacrylates de méthyle par leur densité, celle-ci étant susceptible de varier notamment en fonction de la taille de la cavité sphérique desdites particules.

Dans le cadre de la présente invention, cette densité est appréciée selon le protocole suivant, dit de la densité tassée :

On verse m=40 g de poudre dans une éprouvette graduée ; puis on place l'éprouvette sur l'appareil STAV 2003 de chez STAMPF VOLUMETER ; l'éprouvette est ensuite soumise à 1500 tassements ; puis on mesure directement sur l'éprouvette le volume final Vf de poudre tassée. La densité tassée est déterminée par le rapport m/Vf, en l'occurrence 40/Vf (Vf étant exprimé en cm³ et m en g).

En particulier, la densité des particules de polyméthacrylate de méthyle utilisables selon l'invention peut varier de 0,3 à 0,95, notamment de 0,45 à 0,80, et plus particulièrement de 0,5 à 0,75.

Selon un mode de réalisation particulier de l'invention, la composition selon l'invention comprend au moins deux polyméthacrylates de méthyle différents par leur densité.

Selon cette variante de l'invention, la composition peut comprendre deux types de particules de polyméthacrylate de méthyle dont la différence de densité est d'au moins 0,12, notamment d'au moins 0,15, en particulier d'au moins 0,18, et plus particulièrement d'au moins 0,22.

A titre illustratif, une composition selon l'invention peut associer un polyméthacrylate de méthyle de densité 0,5 à un second polyméthacrylate de méthyle de densité 0,75.

Les polyméthacrylates de méthyle de densités différentes peuvent être associés dans des proportions pondérales variant respectivement de 40 à 60 %, notamment de 45 à 55 %, et en particulier de 48 à 52 % en poids, par rapport au poids total de polyméthacrylates de méthyle, voire dans un rapport pondéral de 1.

Sans vouloir être lié à une quelconque théorie, il semble que les poudres de polyméthacrylate de méthyle de densité supérieure à 0,5 soient plus particulièrement avantageuses pour prolonger les qualités de glissant de la composition dans le temps. Quant aux particules de polyméthacrylate de méthyle de densité inférieure ou égale à 0,5, elles sembleraient pour leur part plus avantageuses en terme de maniabilité de ladite composition. En d'autres termes, le fond de teint se met plus rapidement en place.

Selon une variante particulière de l'invention, la teneur en polyméthacrylate de méthyle de densité supérieure est prédominante par rapport à la teneur en polyméthacrylate de méthyle de densité inférieure.

En particulier, les particules de polyméthacrylate de méthyle de densité supérieure peuvent être des particules pleines, et les particules de polyméthacrylate de méthyle de densité inférieure peuvent être des particules creuses.

Les polyméthacrylates de méthyle selon l'invention peuvent être présents dans la composition en une quantité allant de 1 à 10 % en poids, en particulier de 2 à 7 % et plus particulièrement de 2,5 à 5,5% en poids par rapport au poids total de la composition.

A titre représentatif et non limitatif des polyméthacrylates de méthyle convenant à l'invention, on peut notamment citer les particules de polyméthyméthacrylate commercialisées par la société WACKHERR sous la dénomination Covabead LH 85 et celles commercialisées par la société NIHON JUNYAKU sous la dénomination JURYMER MB1.

Dans le cadre de la présente invention, les polyméthacrylates de méthyle sont avantageusement formulés dans une phase grasse liquide comprenant au moins une huile volatile.

### Diméthicone copolyol

La composition selon l'invention comprend au moins un diméthicone copolyol.

Ce diméthicone copolyol est un polydiméthyl méthyl siloxane oxypropyléné et/ou oxyéthyléné. Il ne contient pas de groupement alkyle à longue chaîne de 8 atomes de carbone ou plus, notamment en C₈-C₂₂.

On peut notamment utiliser comme diméthicone copolyol ceux répondant à la formule (I) suivante: 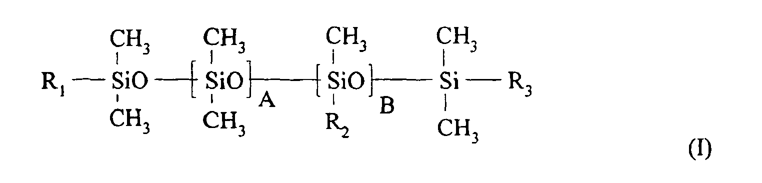 dans laquelle :
- R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, avec au moins un radical R₁, R₂ ou R₃ n'étant pas un radical alkyle et R₄ étant un hydrogène, un radical alkyle en C₁-C₃ ou un radical acyle en C₂-C₄;
- A est un nombre entier allant de 0 à 200 ;
- B est un nombre entier allant de 0 à 50 ; à la condition que A et B ne soient pas égaux à zéro en même temps ;
- x est un nombre entier allant de 1 à 6 ;
- y est un nombre entier allant de 1 à 30 ; et
- z est un nombre entier allant de 0 à 5.

Selon un mode de réalisation particulier de l'invention, dans le composé de formule (I), R₁ = R₃ = radical méthyle, x est un nombre entier allant de 2 à 6 et y est un nombre entier allant de 4 à 30. R₄ est en particulier un hydrogène.

On peut citer, à titre d'exemple de composés de formule (I), les composés de formule (II) : 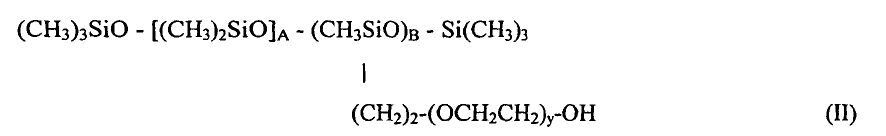 dans laquelle A est un nombre entier allant de 20 à 105, B est un nombre entier allant de 2 à 10 et y est un nombre entier allant de 10 à 20.

On peut également citer à titre d'exemple de composés siliconés de formule (I), les composés de formule (III) :

HO - (CH₂CH₂O)_{y}-(CH₂)₃- [(CH₃)₂SiO]_{A'}- [(CH₃)₂Si]- (CH₂)₃- (OCH₂CH₂)_{y}- OH (III)

dans laquelle A' et y sont des nombres entiers allant de 10 à 20.

On peut notamment utiliser comme diméthicone copolyol ceux vendus sous les dénominations DC 5329, DC 7439-146, DC 2-5695, Q4-3667 par la société DOW CORNING ; KF-6013, KF-6015, KF-6016, KF-6017 par la société SHIN-ETSU.

Les composés DC 5329, DC 7439-146, DC 2-5695 sont des composés de formule (II) où respectivement A est 22, B est 2 et y est 12 ; A est 103, B est 10 et y est 12 ; A est 27, B est 3 et y est 12.

Le diméthicone copolyol peut être présent dans la composition selon l'invention en une teneur allant de 5 % à 10 % en poids, notamment allant de 5 % à 8 % en poids, et en particulier allant de 5 % à 7 % en poids, par rapport au poids total de la composition.

### Alkyl diméthicone copolyol

Selon le second aspect de l'invention, la composition contient au moins un alkyle C₈-C₂₂ diméthicone copolyol.

L'alkyl C₈-C₂₂ diméthicone copolyol présent dans la composition selon l'invention peut être en particulier un poly méthyl alkyl(C₈-C₂₂) diméthyl méthyl siloxane oxypropyléné et/ou oxyéthyléné.

L'alkyl C₈-C₂₂ diméthicone copolyol est avantageusement un composé de formule (IV) suivante : 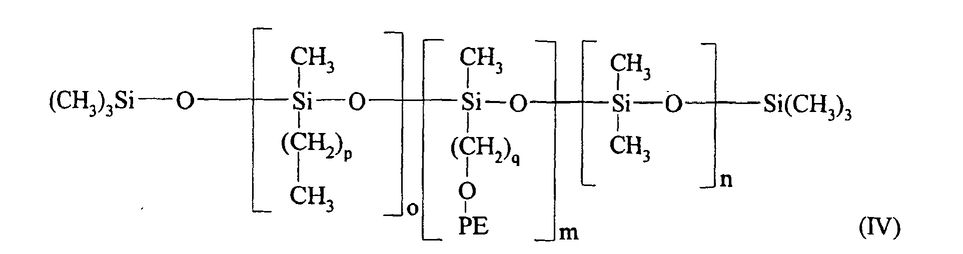 dans laquelle:
- PE représente (-C₂H₄O)ₓ-(C₃H₆O)_{y}-R, R étant choisi parmi un atome d'hydrogène et un radical alkyle de 1 à 4 atomes de carbone, x allant de 0 à 100 et y allant de 0 à 80, x et y n'étant pas simultanément 0 ;
- m allant de 1 à 40 ;
- n allant de 10 à 200 ;
- o allant de 1 à 100 ;
- p allant de 7 et 21 ; et
- q allant de 0 à 4,
et en particulier :
- R=H;
- m = 1 à 10 ;
- n = 10 à 100 ;
- o = 1 à 30 ;
- p = 15 ; et
- q=3

Comme alkyl C₈-C₂₂ diméthicone copolyol convenant à l'invention, on peut en particulier citer le cétyl diméthicone copolyol comme le produit commercialisé sous la dénomination Abil EM-90 par la société GOLDSCHMIDT.

L'alkyl C₈-C₂₂ diméthicone copolyol peut être présent dans la composition selon l'invention en une teneur allant de 0,5 à 2 % en poids, et en particulier allant de 0,5 à 1,5 % en poids, par rapport au poids total de la composition.

### Phase grasse

Par « phase grasse liquide », on entend tout milieu non aqueux liquide à température ambiante (25 °C) et pression atmosphérique (760 mm de Hg), composé d'un ou plusieurs corps gras liquides à température ambiante. Cette phase grasse liquide peut contenir une phase organique liquide volatile et/ou une phase organique liquide non volatile.

Par « phase organique volatile », on entend tout milieu non aqueux susceptible de s'évaporer de la peau, en moins d'une heure à température ambiante et pression atmosphérique. Cette phase volatile comporte notamment des huiles ayant une pression de vapeur, à température ambiante (25 °C) et pression atmosphérique (760 mm Hg) allant de 0,13 Pa à 40 000 Pa (10⁻³ à 300 mm de Hg), notamment allant de 1,3 Pa à 13 000 Pa (0,01 à 100 mm de Hg), et en particulier allant de 1,3 Pa à 1300 Pa (0,01 à 10 mm de Hg).

En outre, l'huile volatile a généralement un point d'ébullition, mesuré à pression atmosphérique, allant de 150 °C à 260 °C, et notamment allant de 170°Cà250°C.

Avantageusement, la phase organique volatile contient une ou plusieurs huiles organiques volatiles dont le point éclair va de 30 °C à 102 °C, en particulier de 40 °C à 55 °C, et notamment de 40 °C à 50 °C et leurs mélanges.

Par « phase organique non volatile », on entend tout milieu susceptible de rester sur la peau pendant plusieurs heures. Une phase organique liquide non volatile a en particulier une pression de vapeur à température ambiante et pression atmosphérique, non nulle inférieure à 0,001 mm de Hg (0,13 Pa).

La phase grasse liquide de l'émulsion selon l'invention contient au moins une huile hydrocarbonée volatile, qui selon le premier aspect de l'invention est au moins de l'isododécane.

Par huile hydrocarbonée, on entend une huile formée essentiellement, voire constituée, d'atomes de carbone et d'hydrogène, et éventuellement d'atomes d'oxygène, d'azote, et ne contenant pas d'atome de silicium ou de fluor. Elle peut contenir des groupes alcool, ester, éther, acide carboxylique, amine et/ou amide.

Comme huile volatile utilisable dans l'invention, on peut citer les huiles volatiles hydrocarbonées ayant de 8 à 16 atomes de carbone, linéaires ou ramifiées, et leurs mélanges et notamment les alcanes ramifiés en C₈-C₁₆ comme les isoalcanes (appelées aussi isoparaffines) en C₈-C₁₆, l'isododécane, l'isohexadécane et par exemple les huiles vendues sous les noms commerciaux d'Isopars^{®} ou de Permethyls^{®}, les esters ramifiés en C₈-C₁₆ comme le néo pentanoate d'iso-hexyle et leurs mélanges. On utilise en particulier l'isododécane.

L'huile hydrocarbonée volatile, et notamment l'isododécane, peut être présente en une teneur allant de 5 % à 25 % en poids, notamment allant de 10 % à 20 % en poids, et en particulier allant de 10 % à 15 % en poids, par rapport au poids total de la composition.

L'émulsion selon l'invention peut comprendre une huile volatile additionnelle, c'est-à-dire distincte de l'huile volatile requise selon l'invention, qui peut être choisie parmi les huiles volatiles hydrocarbonées, avec celle-ci différente de l'isododécane lorsque la composition comprend de l'isododécane, les huiles volatiles siliconées comportant éventuellement des groupes alkyles ou alkoxy pendants, ou en bout de chaîne siliconée, les huiles volatiles fluorées, et leurs mélanges.

Comme huile siliconée volatile utilisable dans l'invention, on peut citer les huiles de silicones linéaires, ramifiées ou cycliques ayant une viscosité à température ambiante inférieure à 8 mm²/s et ayant notamment de 2 à 7 atomes de silicium, ces silicones comportant éventuellement des groupes alkyle ou alkoxy ayant de 1 à 10 atomes de carbone pendants ou au bout de chaque silicone. Comme huile de silicone volatile utilisable dans l'invention, on peut citer notamment l'octaméthyl cyclotétrasiloxane, le décaméthyl cyclopentasiloxane, le dodécaméthyl cyclohexasiloxane, l'heptaméthylhexyl trisiloxane, l'heptaméthyloctyl trisiloxane, l'hexaméthyl disiloxané, l'octaméthyl trisiloxane, le décaméthyl tétrasiloxane, le dodécaméthyl pentasiloxane et leurs mélanges. En revanche, l'huile de silicone cyclotétrasiloxane ne sera pas considérée selon l'invention notamment en raison de son défaut d'innocuité.

L'huile volatile fluorée n'a généralement pas de point éclair.

Comme huile volatile fluorée, on peut citer le nonafluoroéthoxybutane, le nonafluorométhoxybutane, le décafluoropentane, le tétradécafluorohexane, le dodécafluoropentane, et leurs mélanges.

Avantageusement, l'huile volatile additionnelle représente de 20 à 32 % en poids, notamment de 20 à 30 % en poids, en particulier de 22 à 26 % en poids, par rapport au poids total de la composition.

La composition peut comprendre, en outre, au moins une huile non volatile.

Comme huiles non volatiles utilisables dans l'invention, on peut citer les huiles hydrocarbonées d'origine minérale ou synthétique telles que les hydrocarbures linéaires ou ramifiés comme l'huile de paraffine ou ses dérivés, l'huile de vaseline, les polydécènes, le polyisobutène hydrogéné tel que le Parléam commercialisé par la société NIPPON OIL FATS, le squalane d'origine synthétique ou végétale ; les huiles d'origine animale comme l'huile de vision, de tortue, le perhydrosqualène ; les huiles d'origine végétale hydrocarbonées à forte teneur en triglycérides constitués d'esters d'acides gras et de glycérol dont les acides gras peuvent avoir des longueurs de chaînes variées, ces dernières pouvant être linéaires ou ramifiées, saturées ou insaturées, notamment les triglycérides d'acide gras notamment de 4 à 22 atomes de carbone, comme les triglycérides des acides heptanoïque, octanoïque, et des acides caprique/caprylique ou bien encore les triglycérides hydroxylés, tels que l'huile d'amande douce, de calophyllum, de palme, de pépins de raisin, de sésame, d'arara, de colza, de tournesol, de coton, d'abricot, de ricin, de luzerne, de courge, de cassis, de macadamia, de rosier muscat, de noisette, d'avocat, de jojoba, d'olive, de germes de céréales (maïs, blé, orge, seigle), de beurre de karité ; des esters d'acides gras, en particulier de 4 à 22 atomes de carbone, et notamment d'acide octanoïque, d'acide heptanoïque, d'acide lanolique, d'acide oléique, d'acide laurique, d'acide stéarique comme le dioctanoate de propylène glycol, le monoisostéarate de propylène glycol, le polyglycéryl 2 diisostéarate, le diheptanoate de néopentylglycol ; les esters de synthèse de formule R₁COOR₂ dans laquelle R₁ représente le reste d'un acide gras supérieur linéaire ou ramifié comportant de 7 à 40 atomes de carbone et R₂ représente une chaîne hydrocarbonée ramifiée contenant de 3 à 40 atomes de carbone, comme par exemple l'huile de Purcellin (octanoate de cétostéaryle), l'isononanoate d'isononyle, le benzoate d'alcool en C₁₂ à C₁₅, le palmitate d'éthyl 2-hexyle, le stéarate d'octyl-2 dodécyle, l'érucate d'octyl-2 dodécyle, l'isostéarate d'isostéaryle, le benzoate d'octyl-2 dodécyle, des octanoates, décanoates ou ricinoléates d'alcools ou de polyalcools, le myristate d'isopropyle, le palmitate d'isopropyle, le stéarate de butyle, le laurate d'hexyle, l'adipate de diisopropyle, le palmitate de 2-éthyl-hexyle, le laurate de 2-hexyl-décyle, le palmitate de 2-octyl-décyle, le myristate de 2-octyldodécyle, le succinate de 2-diéthyl-hexyle, le malate de diisostéaryle, le triisostéarate de glycérine ou de diglycérine ; les esters hydroxylés comme le lactate d'isostéaryle, l'octylhydroxystéarate, l'hydroxystéarate d'octyldodécyle, le diisostéarylmalate, le citrate de triisocétyle ; le diisononanoate de diéthylèneglycol ; les esters du pentaérythritol ; les esters d'acides aromatiques et d'alcools comprenant 4 à 22 atomes de carbone, notamment le trimellitate de tridécyle ; les acides gras supérieurs en C₈-C₂₆ tels que l'acide oléique, l'acide linoléique, l'acide linolénique ou l'acide isostéarique ; les alcools gras supérieurs en C₈-C₂₆ tels que l'alcool oléique, l'alcool linoléique ou linolénique, l'alcool isostéarique ou l'octyl dodécanol ; les éthers de synthèse à au moins 7 atomes de carbone, les huiles siliconées telles que les polydiméthylsiloxanes (PDMS) liquides à température ambiante, linéaires, éventuellement phénylés tels que les phényltriméthicones, les phényl triméthylsiloxy diphénylsiloxanes, les diphényl diméthicones, les diphényl méthyldiphényl trisiloxanes, les 2-phényléthyl triméthylsiloxysilicates liquides, éventuellement substitués par des groupements aliphatiques et/ou aromatiques comme les groupes alkyle, alkoxy ou phényle, pendant et/ou en bout de chaîne siliconée, groupements ayant de 2 à 24 atomes de carbone et éventuellement fluorés, ou par des groupements fonctionnels tels que des groupements hydroxyle, thiol et/ou amine ; les polysiloxanes modifiés par des acides gras, des alcools gras ou des polyoxyalkylènes comme les diméthicones copolyols ou les alkylméthicones copolyols ; les silicones fluorées liquides ; ou encore les triglycérides des acides caprylique/caprique comme ceux vendus par la société STEARINERIES Dubois ou ceux vendus sous les dénominations Miglyol 810, 812 et 818 par la société DYNAMIT NOBEL, et leurs mélanges.

La composition peut comprendre une ou plusieurs huiles non volatiles en une teneur allant de 0,1 à 12 % en poids, et notamment de 1 à 5 % en poids, par rapport au poids total de la composition.

La composition selon l'invention peut également comprendre au moins une cire, au moins une gomme et/ou au moins un corps gras pâteux, d'origine végétale, animale, minérale ou de synthèse, siliconé ou non.

Par « cire » au sens de la présente invention, on entend un composé gras lipophile, solide à température ambiante (25 °C), à changement d'état solide/liquide réversible, ayant une température de fusion supérieure à 30 °C pouvant aller jusqu'à 200 °C, une dureté supérieure à 0,5 MPa, et présentant à l'état solide une organisation cristalline anisotrope. En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.

Les cires, au sens de la présente invention, peuvent être hydrocarbonées, siliconées et/ou fluorées, comportant éventuellement des fonctions ester ou hydroxyle. Elles sont notamment d'origine naturelle comme la cire d'abeilles éventuellement modifiée, la cire de Carnauba, de Candellila, d'Ouricoury, du Japon, de fibres de liège ou de canne à sucre, la cérésine, les cires de paraffine, de lignite, les cires microcristallines, la cire de lanoline, la cire de Montan, les ozokérites, les huiles hydrogénées comme l'huile de jojoba hydrogénée ou copolymérisation de l'éthylène, les cires obtenues par synthèse de Fischer-Tropsch, les esters d'acides gras et les glycérides concrets à 45 °C, les cires de silicone comme les alkyle, alkoxy et/ou esters de poly(di)méthylsiloxane solide à 45 °C, ayant de 10 à 45 atomes de carbone, certains acides gras comme l'acide stéarique, myristique, ou béhénique, et leurs mélanges.

La cire peut représenter de 0,01 à 10 % en poids, notamment de 0,1 à 5 % en poids, par rapport au poids total de la composition. Selon un mode de réalisation, la composition peut être exempte de cires.

Par « composé pâteux » au sens de l'invention, on entend un composé ayant un point de fusion allant de 25 à 60 °C, de préférence de 30 à 45 °C et une dureté allant de 0,001 à 0,5 MPa, de préférence de 0,005 à 0,4 MPa.

A titre d'exemple de corps gras pâteux, on peut citer les PDMS ayant des chaînes pendantes du type alkyle ou alkoxy ayant de 8 à 24 atomes de carbone comme le stéaryl diméthicone et notamment ceux vendus par DOW CORNING sous les références DC2503 ou DC05514 ; les esters d'alcool gras ou d'acide gras ayant de 20 à 25 atomes de carbone (un point de fusion notamment de 20 à 35 °C et/ou une viscosité à 40 °C allant de 0,1 à 40 Pa.s) comme les esters du cholestérol tels que les triglycérides d'origine végétale hydrogénés comme par exemple l'huile de ricin hydrogénée vendue sous le nom « Thixinr » par la société RHEOX, le polylaurate de vinyle, le propionate d'arachidyle, le triisostéaryl or cétyl citrate, le copolymère PVP/Eicosène ; les lanolines d'isopropyle, ayant une viscosité de 10 à 25 Pa.s, de préférence de 19 à 25 Pa.s et/ou un point de fusion de 25 à 45 °C et leurs mélanges.

La composition de l'invention peut également comprendre au moins une alkyl, alkoxy ou phényl-diméthicone telle que, par exemple le produit vendu sous la dénomination de « Abil wax 2440^{®} » par la société GOLDSCHMIDT.

### Phase aqueuse

La composition selon l'invention comprend au moins une phase aqueuse contenant de l'eau. L'eau peut être une eau florale telle que l'eau de bleuet et/ou une eau minérale telle que l'eau de VITTEL, l'eau de LUCAS ou l'eau de LA ROCHE POSAY et/ou une eau thermale.

Cette phase aqueuse peut également comprendre des solvants organiques miscibles à l'eau (25 °C) comme par exemple les alcools primaires tels que l'éthanol et l'isopropanol, les glycols tels que le glycérol, le propylène glycol, le butylène glycol, le dipropylène glycol, le diéthylène glycol, les éthers de glycol, les alkyl en C₁-C₄ éthers de mono-, di- ou tri- propylène glycol, mono-, di- ou tri- éthylène glycol, et leurs mélanges.

La phase aqueuse peut comprendre en outre des agents de stabilisation, par exemple le chlorure de sodium, le dichlorure de magnésium et le sulfate de magnésium.

Elle peut également incorporer tout composé hydrosoluble ou hydrodispersible compatible avec une phase aqueuse tels que des gélifiants, des polymères filmogènes, des épaississants, des tensioactifs et leurs mélanges.

Selon un mode de réalisation particulier, la phase aqueuse, et notamment l'eau, peut être présente dans la composition selon l'invention en une teneur allant de 30 à 50 % en poids, notamment de 35 à 45 % en poids, par rapport au poids total de la composition.

### Epaississant

L'émulsion selon l'invention peut comprendre, en outre, un agent épaississant de la phase grasse. L'agent épaississant peut être choisi parmi :
- les argiles organomodifiées qui sont des argiles traitées par des composés choisis notamment parmi les amines quaternaires, les amines tertiaires. Comme argiles organomodifiées, on peut citer les bentonites organomodifiées telles que celles vendues sous la dénomination "Bentone 34" par la société RHEOX, les hectorites organomodifiées telles que celles vendues sous la dénomination "Bentone 27", "Bentone 38" par la société RHEOX,
- la silice pyrogénée hydrophobe, qui est une silice pyrogénée modifiée chimiquement en surface par réaction chimique générant une diminution du nombre de groupes silanol. On peut notamment substituer des groupes silanol par des groupements hydrophobes.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812^{®}" par la société DEGUSSA, "CAB-O-SIL TS-530^{®}" par la société CABOT,
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL R972^{®}", "AEROSIL R974^{®}" par la société DEGUSSA, "CAB-O-SIL TS-610^{®}", "CAB-O-SIL TS-720^{®}" par la société CABOT.

La silice pyrogénée présente de préférence une taille de particules pouvant être nanométrique à micrométrique, par exemple allant d'environ de 5 à 200 nm.

L'agent épaississant de la phase grasse peut être présent en une teneur allant de 0,1 à 5 % en poids, et notamment de 0,4 à 3 % en poids par rapport au poids total de la composition.

### Charges

La composition selon l'invention peut comprendre en outre au moins une charge additionnelle, différente des poudres de polyméthacrylates de méthyle.

Par « charge », on entend toute particule incolore choisie parmi les charges minérales ou organiques, lamellaires, sphériques ou oblongues, chimiquement inerte dans la composition.

On peut citer le talc, le mica, la silice, le kaolin, les poudres de polyamide comme le Nylon^{®}, les poudres de poly-β-alanine et de polyéthylène, les poudres de polymères de tétrafluoroéthylène (Téflon^{®}), la lauroyl-lysine, l'amidon, le nitrure de bore, les microsphères creuses polymériques telles que celles de chlorure de polyvinylidène/acrylonitrile telles que l'Expancel^{®} (NOBEL INDUSTRIE), les particules de polymère acrylique, notamment de copolymère d'acide acrylique comme le Polytrap^{®} (DOW CORNING), les poudres de polyuréthane, les microbilles de résine de silicone (Tospearls^{®} de TOSHIBA, par exemple), le carbonate de calcium précipité, le dicalcium phosphate, le carbonate ou l'hydrocarbonate de magnésium, l'hydroxyapatite, les microsphères de silice creuses (Silica Beads^{®} de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges. Ces charges peuvent être traitées ou non en surface notamment pour les rendre lipophiles.

Les charges peuvent être présentes dans la composition selon l'invention en une teneur allant de 0,1 à 10 % en poids, notamment de 0,1 à 7 % en poids, par rapport au poids total de la composition.

### Matières colorantes

La composition peut contenir avantageusement au moins une matière colorante qui peut être choisie parmi les colorants lipophiles, les colorants hydrophiles, les pigments et les nacres habituellement utilisés dans les compositions cosmétiques ou dermatologiques, et leurs mélanges. Cette matière colorante est généralement présente à raison de 0,01 à 40 % en poids, notamment de 1 à 35 % en poids, en particulier de 2 à 25 % en poids, et plus particulièrement de 3 à 20 % en poids par rapport au poids total de la composition.

Les colorants liposolubles sont par exemple le rouge Soudan, le DC Red 17, le DC Green 6, le β-carotène, l'huile de soja, le brun Soudan, le DC Yellow 11, le DC Violet 2, le DC orange 5, le jaune quinoléine. Ils peuvent représenter de 0,1 à 20 % en poids, et en particulier de 0,1 à 6 % en poids, par rapport au poids total de la composition.

Les pigments peuvent être blancs ou colorés, minéraux et/ou organiques, enrobés ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D&C, et les laques à base de carmin de cochenille, de baryum, strontium, calcium, aluminium. Les pigments peuvent représenter de 0,1 à 40 % en poids, notamment de 1 à 35 % en poids, et en particulier de 2 à 25 % en poids, par rapport au poids total de la composition.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth. Ils peuvent représenter de 0,1 à 20 % en poids, et en particulier de 0,1 à 15 % en poids, par rapport au poids total de la composition.

Avantageusement, l'émulsion selon l'invention comprend des pigments enrobés hydrophobes. Les pigments enrobés hydrophobes sont des pigments (tels que ceux cités précédemment) traités en surface avec un agent hydrophobe pour les rendre compatibles avec la phase grasse de l'émulsion, notamment pour qu'ils aient une bonne mouillabilité avec les huiles de la phase grasse de manière à ce que ces pigments traités soient bien dispersés dans la phase grasse.

L'agent de traitement hydrophobe peut être choisi parmi les silicones comme les méthicones, les diméthicones, les perfluoroalkylsilanes ; les acides gras comme l'acide stéarique ; les savons métalliques comme le dimyristate d'aluminium, le sel d'aluminium du glutamate de suif hydrogéné, les perfluoroalkyl phosphates, les perfluoroalkyl silanes, les perfluoroalkyl silazanes, les polyoxydes d'hexafluoropropylène, les polyorganosiloxanes comprenant des groupes perfluoroalkylles perfluoropolyéthers, les acides aminés ; les acides aminés N-acylés ou leurs sels ; la lécithine, le trisostéaryle titanate d'isopropyle, et leurs mélanges.

Les acides aminés N-acylés peuvent comprendre un groupe acyle ayant de 8 à 22 atomes de carbones, comme par exemple un groupe 2-éthyl hexanoyle, caproyle, lauroyle, myristoyle, palmitoyle, stéaroyle, cocoyle. Les sels de ces composés peuvent être les sels d'aluminium, de magnésium, de calcium, de zirconium, de zinc, de sodium, de potassium. L'acide aminé peut être par exemple la lysine, l'acide glutamique, l'alanine.

Le terme « alkyle » mentionné dans les composés agents hydrophobes cités précédemment désigne notamment un groupe alkyle ayant de 1 à 30 atomes de carbone, de préférence ayant de 5 à 16 atomes de carbone.

Des pigments traités hydrophobes sont notamment décrits dans la demande EP-A-1086683.

### Rhéologie

Avantageusement, la composition selon l'invention peut posséder une viscosité, mesurée à 25 °C, à une vitesse de cisaillement de 200 min⁻¹ (200 tours par minute, soit une fréquence de 50 Hz), allant de 0,15 à 0,6 Pa.s (1,5 à 6 poises), et notamment allant de 0,25 à 0,45 Pa.s (2,5 à 4,5 poises). Une telle viscosité permet une application facile l'émulsion et d'obtenir un maquillage homogène, uniforme et sans trace. La viscosité est mesurée à 25 °C avec un viscosimètre CONTRAVES type TV équipé d'un mobile n°2, la mesure étant effectuée après 10 minutes de rotation du mobile (temps au bout duquel on observe une stabilisation de la viscosité et de la vitesse de rotation du mobile), à un cisaillement de 200 min⁻¹.

### Additifs

La composition de l'invention peut comprendre, en outre, tout additif complémentaire usuellement utilisé dans le domaine concerné, tel que des antioxydants, des conservateurs, des neutralisants, des gélifiants lipophiles, des gélifiants de phase aqueuse, des dispersants, des actifs cosmétiques, des parfums, des agents anti-radicaux libres, des séquestrants, des agents filmogènes, et leurs mélanges. Ces additifs peuvent être présents dans la composition à raison de 0,0005 à 20 % en poids, et en particulier de 0,001 à 10 % en poids, par rapport au poids total de la composition.

Comme actif cosmétique utilisable dans l'invention, on peut citer les vitamines A, E, C, B₃, F, les provitamines comme le D-panthénol, les actifs apaisants comme l'a-bisabolol, l'aloe vera, l'allantoïne, les extraits de plantes ou les huiles essentielles, les agents protecteurs ou restructurants comme les céramides, les actifs « fraîcheur » comme le menthol et ses dérivés, les émollients, les hydratants, les actifs antirides, les acides gras essentiels, les filtres solaires, et leurs mélanges.

Bien entendu, l'homme du métier veillera à choisir les éventuels additifs complémentaires et/ou leur quantité de telle manière que les propriétés avantageuses de la composition selon l'invention ne soient pas ou substantiellement pas, altérées par l'adjonction envisagée.

La composition selon l'invention peut être fabriquée par les procédés connus, généralement utilisés dans le domaine cosmétique.

La composition selon l'invention peut être une composition de maquillage de la peau, comme un fond de teint, un produit anti-cernes, une crème teintée, une composition de maquillage du corps, et en particulier un fond de teint à appliquer sur le visage et/ou le cou.

### Exemple

L'exemple figurant ci-après est présenté à titre illustratif et non limitatif du domaine de l'invention.

### Exemple 1

| Formulation de fond de teint | | % en poids |
|---|---|---|
| - Butylène-1,3 glycol | | 10,00 |
| - Hectorite modifiée par chlorure de di-stéaryl di-méthyl ammonium (commercialisée sous le nom BENTONE 38 V par ELEMENTIS) | | 1,60 |
| - Conservateurs | | 0,90 |
| - Eau | qsp | 100,00 |
| - Cyclopenta diméthylsiloxane | | 11,36 |
| - Néopentanoate d'iso-stéaryle | | 0,50 |
| - Chlorure de sodium | | 0,70 |
| - Isododécane | | 13,00 |
| - Cyclohexadiméthylsiloxane | | 8,00 |
| - Poly diméthylsiloxane (DC 200 Fluid 5 est commercialisé par DOW CORNING) | | 2,00 |
| - Cétyl diméthicone copolyol (commercialisé sous la dénomination ABIL EM90 par GOLDSCHMIDT) | | 0,80 |
| - Isostéarate polyglycérol | | 0,60 |
| - Isoeicosane | | 2,00 |
| - Laurate d'hexyle | | 0,60 |
| - Microsphères creuses de polyméthacrylate de méthyle (commercialisées sous la dénomination COVABEAD LH85 par WACKHERR) | | 2,00 |
| - Poudre de polyméthylméthacrylate (commercialisée sous la dénomination JURYMER MB1 par N IHON JUNYAKU) | | 2,00 |
| - Poly diméthylsiloxane oxyéthyléné (commercialisé sous la dénomination KF-6017 de SHIN ETSU) | | 4,48 |
| - Nacre | | 1,00 |
| - Oxyde de fer jaune enrobé de phosphate de perfluoroalkyle en dispersion dans décaméthyl cyclopentasiloxane/diméthicone copolyol (commercialisé sous la dénomination FASODYF par KOBO) | | 2,40 |
| - Oxyde de fer brun enrobé de phosphate de perfluoroalkyle en dispersion dans cyclométhicone/diméthyl polysiloxane copolyol (commercialisé sous la dénomination FA50DRF par KOBO) | | 0,93 |
| - Oxyde de fer noir enrobé de phosphate de perfluoroalkyle en dispersion dans décaméthyl cyclopentasiloxane/diméthicone copolyol (commercialisé sous la dénomination FA65DBF par KOBO) | | 0,44 |
| - Oxyde de titane traité alumine enrobé de phosphate de perfluoroalkyle dans décaméthyl cyclopentasiloxane/diméthicone copolyol (commercialisé sous la dénomination FA65DF par KOBO) | | 9,54 |

Ce fond de teint s'applique facilement sur la peau, s'avère posséder des qualités de glissant sur la peau avantageuses et peut être facilement travaillé pour bien répartir de manière homogène le maquillage sur le visage.

## Revendications

1. Composition cosmétique fluide sous forme d'une émulsion eau-dans-huile comprenant une phase grasse liquide, une phase aqueuse et un diméthicone copolyol, **caractérisée en ce qu'**elle comprend des particules sphériques solides de polyméthacrylate de méthyle et que la phase grasse liquide comprend de l'isododécane, ladite composition étant exempte de cyclotétrasiloxane.

2. Composition cosmétique fluide sous forme d'une émulsion eau-dans-huile comprenant une phase grasse liquide, une phase aqueuse, un diméthicone copolyol et un alkyl C₈-C₂₂ diméthicone copolyol, **caractérisée en ce qu'**elle comprend des particules sphériques solides de polyméthacrylate de méthyle et que la phase grasse liquide comprend une huile hydrocarbonée volatile, ladite composition étant exempte de cyclotétrasiloxane.

3. Composition selon l'une des revendications 1 ou 2, **caractérisée en ce que** les particules solides de polyméthacrylate de méthyle possèdent une densité variant de 0,3 à 0,95, notamment de 0,45 à 0,80, et plus particulièrement de 0,5 à 0,75.

4. Composition selon l'une des revendications 1 ou 2, **caractérisée en ce qu'**elle comprend au moins deux polyméthacrylates de méthyle différents en terme de densité.

5. Composition cosmétique fluide sous forme d'une émulsion eau-dans-huile comprenant une phase grasse liquide, une phase aqueuse et un diméthicone copolyol, **caractérisée en ce qu'**elle comprend des particules solides de polyméthacrylate de méthyle avec au moins deux polyméthacrylates de méthyle différents en terme de densité et **en ce que** la phase grasse liquide comprend de l'isododécane, ladite composition étant exempte de cyclotétrasiloxane.

6. Composition selon la revendication précédente 4 ou 5, **caractérisée en ce qu'**elle associe deux types de particules de polyméthacrylate de méthyle dont la différence de densité est d'au moins 0,12, notamment d'au moins 0,15, en particulier d'au moins 0,18, et plus particulièrement d'au moins 0,22.

7. Composition selon l'une quelconque des revendications 4 à 6, **caractérisée en ce que** les particules de polyméthacrylates de méthyle différents en terme de densité sont présentes dans des proportions pondérales variant respectivement de 40 à 60 % en poids, notamment de 45 à 55 %, et en particulier de 48 à 52 % par rapport au poids total de polyméthacrylates de méthyle.

8. Composition selon la revendication 7, **caractérisée en ce que** la teneur en polyméthacrylate de méthyle de densité supérieure est prédominante par rapport à la teneur en polyméthacrylate de méthyle de densité inférieure.

9. Composition selon l'une quelconque des revendications précédentes, **caractérisée en ce qu'**elle comprend de 1 à 10 % en poids, notamment de 2 à 7 % en poids, et plus particulièrement de 2,5 à 5,5 % en poids de polyméthacrylate de méthyle, par rapport au poids total de ladite composition.

10. Composition selon l'une quelconque des revendications 1 à 9, **caractérisée en ce que** le diméthicone copolyol est un composé de formule (I) suivante : 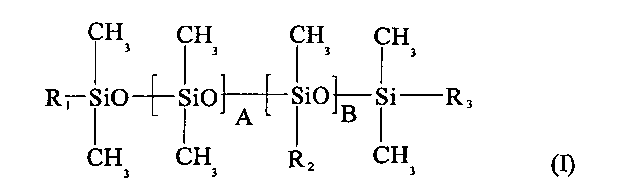 dans laquelle:
- R₁, R₂, R₃, indépendamment les uns des autres, représentent un radical alkyle en C₁-C₆ ou un radical -(CH₂)ₓ - (OCH₂CH₂)_{y} - (OCH₂CH₂CH₂)_{z} - OR₄, avec au moins un radical R₁, R₂ ou R₃ n'étant pas un radical alkyle et R₄ étant un hydrogène, un radical alkyle en C₁-C₃ ou un radical acyle en C₂-C₄ ;
- A est un nombre entier allant de 0 à 200 ;
- B est un nombre entier allant de 0 à 50 ; à la condition que A et B ne soient pas égaux à zéro en même temps ;
- x est un nombre entier allant de 1 à 6 ;
- y est un nombre entier allant de 1 à 30 ; et
- z est un nombre entier allant de 0 à 5.

11. Composition selon la revendication 10, **caractérisée en ce que** R₁ = R₃ = radical méthyle, x est un nombre entier allant de 2 à 6 et y est un nombre entier allant de 4 à 30.

12. Composition selon la revendication 10 ou 11, **caractérisée en ce que** R₄ est un atome d'hydrogène.

13. Composition selon l'une quelconque des revendications 10 à 12, **caractérisée en ce que** le diméthicone copolyol est un composé de formule (II) suivante : 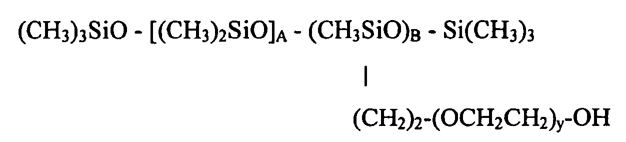 dans laquelle A est un nombre entier allant de 20 à 105, B est un nombre entier allant de 2 à 10 et y est un nombre entier allant de 10 à 20.

14. Composition selon la revendication 10, **caractérisée en ce que** le diméthicone copolyol est un composé de formule (III) suivante :
HO - (CH₂CH₂O)_{y} - (CH₂)₃ - [(CH₃)₂SiO]_{A'} - [(CH₃)₂Si] - (CH₂)₃ - (OCH₂CH₂)_{y}- OH (III)
dans laquelle A' et y sont des nombres entiers allant de 10 à 20.

15. Composition selon l'une quelconque des revendications 10 à 14, **caractérisée en ce que** le diméthicone copolyol est présent en une teneur allant de 5 % à 10 % en poids, notamment allant de 5 % à 8 % en poids, et en particulier allant de 5 % à 7 % en poids, par rapport au poids total de la composition.

16. Composition selon l'une quelconque des revendications 2 et 4 à 15, **caractérisée en ce que** l'alkyl C₈-C₂₂ diméthicone copolyol est un composé de formule (IV) suivante : 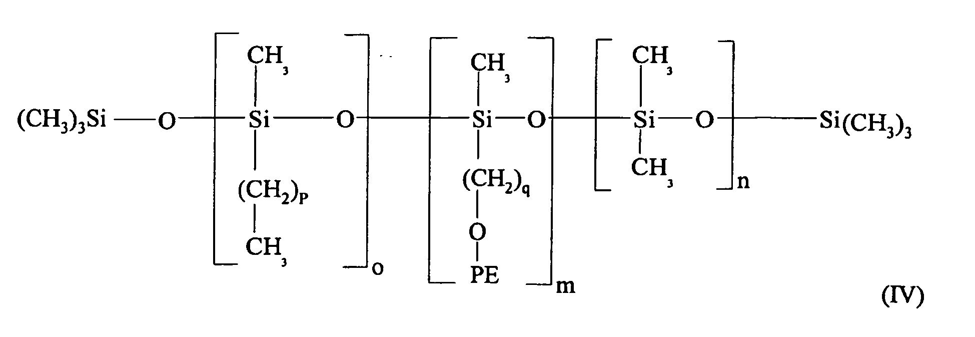 dans laquelle :
- PE représente (-C₂H₄O)ₓ-(C₃H₆O)_{y}-R, R étant choisi parmi un atome d'hydrogène et un radical alkyle de 1 à 4 atomes de carbone, x allant de 0 à 100 et y allant de 0 à 80, x et y n'étant pas simultanément 0 ;
- m va de 1 à 40 ;
- n va de 10 à 200 ;
- o va de 1 à 100 ;
- p va de 7 à 21 ; et
- q va de 0 à 4.

17. Composition selon la revendication 16, **caractérisée en ce que** R = H;
m = 1 à 10 ; n = 10 à 100 : o = 1 à 30 ; p = 15 et q = 3.

18. Composition selon l'une quelconque des revendications 2 et 4 à 17, **caractérisée en ce que** l'alkyl C₈-C₂₂ diméthicone copolyol est le cétyl diméthicone copolyol.

19. Composition selon l'une quelconque des revendications 2 et 4 à 18, **caractérisée en ce que** l'alkyl C₈-C₂₂ diméthicone copolyol est présent en une teneur allant de 0,5 % à 2 % en poids, et en particulier allant de 0,5 % à 1,5 % en poids, par rapport au poids total de la composition.

20. Composition selon l'une quelconque des revendications 2 et 4 à 19, **caractérisée en ce que** l'huile volatile hydrocarbonée est choisie parmi les huiles volatiles hydrocarbonées ayant de 8 à 16 atomes de carbone, linéaires ou ramifiés.

21. Composition selon la revendication 20, **caractérisée en ce que** l'huile hydrocarbonée volatile est choisie parmi l'isododécane, l'isohexadécane, le néo pentanoate d'iso-hexyle et leurs mélanges.

22. Composition selon l'une quelconque des revendications précédentes, **caractérisée en ce que** l'huile hydrocarbonée volatile et notamment l'isododécane, représente de 5 à 25 %, notamment de 10 à 20 % et en particulier de 10 à 15 % en poids, par rapport au poids total de la composition.

23. Composition selon l'une quelconque des revendications précédentes, **caractérisée en ce que** l'isododécane ou l'huile hydrocarbonée volatile est associée à au moins une huile volatile additionnelle distincte choisie parmi les huiles hydrocarbonées, fluorées et/ou siliconées comportant éventuellement des groupes alkyles ou alkoxy, pendants ou en bout de chaîne siliconée.

24. Composition selon la revendication 23, **caractérisée en ce que** l'huile siliconée est une huile silicone linéaire, ramifiée ou cyclique ayant une viscosité à température ambiante inférieure à 8 mm²/s et possédant de 2 à 7 atomes de silicium.

25. Composition selon la revendication 23 ou 24, **caractérisée en ce que** l'huile siliconée est choisie parmi l'octaméthyl cyclotétrasiloxane, le décaméthyl cyclopentasiloxane, le dodécaméthyl cyclohexasiloxane, l'heptaméthylhexyl trisiloxane, l'heptaméthyloctyl trisiloxane, l'hexaméthyl disiloxane, l'octaméthyl trisiloxane, le décaméthyl tétrasiloxane, le dodécaméthyl pentasiloxane et leurs mélanges.

26. Composition selon l'une quelconque des revendications 23 à 25, **caractérisée en ce que** l'huile volatile additionnelle représente de 20 à 32 %, notamment de 20 à 30 %, et plus particulièrement de 22 à 26 % en poids, par rapport au poids total de la composition.

27. Composition selon l'une quelconque des revendications précédentes, **caractérisée en ce que** la composition comprend en outre au moins une huile non volatile.

28. Composition selon la revendication 27, **caractérisée en ce que** l'huile non volatile est choisie parmi les huiles hydrocarbonées d'origine minérale ou synthétique, les huiles d'origine animale, les huiles d'origine végétale hydrocarbonées à forte teneur en triglycérides constitués d'esters d'acides gras et de glycérol, les esters d'acides gras, en particulier comprenant de 4 à 22 atomes de carbone, les esters de synthèse de formule R₁COOR₂ dans laquelle R₁ représente le reste d'un acide gras supérieur linéaire ou ramifié comportant de 7 à 40 atomes de carbone, et R₂ représente une chaîne hydrocarbonée ramifiée contenant de 3 à 40 atomes de carbone, les esters hydroxylés, les esters d'acides aromatiques et d'alcool comprenant 4 à 22 atomes de carbone, les acides gras supérieurs en C₈ à C₂₆, les alcools gras supérieurs en C₈ à C₂₆, les éthers de synthèse à au moins 7 atomes de carbone, les huiles siliconées, les polysiloxanes modifiés par des acides gras, des alcools gras ou des polyoxyalkylènes, les silicones fluorées liquides, les triglycérides des acides caprylique/caprique et leurs mélanges.

29. Composition selon la revendication 27 ou 28 **caractérisée en ce qu'**elle comprend 0,1 à 12 %, et notamment 1 à 5 % par rapport au poids total de la composition en poids d'au moins une huile non volatile.

30. Composition selon l'une quelconque des revendications précédentes, **caractérisée en ce qu'**elle comprend au moins une cire, au moins une gomme et/ou au moins un corps gras pâteux d'origine végétale, animale, minérale ou de synthèse, siliconé ou non.

31. Composition selon l'une quelconque des revendications précédentes, **caractérisée en ce qu'**elle comprend 0,01 à 10 % en poids et notamment 0,1 à 5 % en poids d'au moins une cire par rapport au poids total de la composition.

32. Composition selon l'une quelconque des revendications précédentes, **caractérisée en ce qu'**elle comprend un agent épaississant de la phase grasse liquide.

33. Composition selon la revendication 32, **caractérisée en ce que** l'agent épaississant est choisi parmi les argiles organomodifiés et la silice pyrogénée hydrophobe.

34. Composition selon la revendication 32 ou 33, **caractérisée en ce que** l'agent épaississant de la phase grasse est présent en une teneur allant de 0,1 à 5 % en poids et notamment de 0,4 à 3 % en poids par rapport au poids total de la composition.

35. Composition selon l'une quelconque des revendications précédentes, **caractérisée en ce que** la phase aqueuse comprend au moins un solvant organique miscible à l'eau et/ou des agents de stabilisation et/ou au moins un composé hydrosoluble ou hydrodispersible.

36. Composition selon l'une quelconque des revendications précédentes, **caractérisée en ce que** ladite phase aqueuse est présente à raison de 30 à 50 % en poids et notamment de 35 à 45 % en poids par rapport au poids total de la composition.

37. Composition selon l'une quelconque des revendications précédentes, **caractérisée en ce qu'**elle comprend en outre au moins une charge additionnelle distincte des poudres de polyméthacrylate de méthyle.

38. Composition selon la revendication précédente, **caractérisée en ce que** cette charge est présente à raison de 0,1 à 10 % en poids et en particulier de 0,1 à 7 % en poids par rapport au poids total de la composition.

39. Composition selon l'une quelconque des revendications précédentes, **caractérisée en ce qu'**elle comprend en outre une matière colorante choisie parmi les colorants lipophiles, les colorants hydrophiles, les pigments et les nacres.

40. Composition selon la revendication 39, **caractérisée en ce que** les pigments sont des pigments enrobés hydrophobes.

41. Composition selon la revendication 39 ou 40, **caractérisée en ce que** cette matière colorante est présente à raison de 0,01 à 40 % en poids, notamment de 1 à 35 % en poids, en particulier de 2 à 25 % en poids, et plus particulièrement de 3 à 20 % en poids par rapport au poids total de la composition.

42. Composition selon l'une quelconque des revendications précédentes, **caractérisée en ce qu'**elle se présente sous forme d'une composition de maquillage de la peau, et en particulier d'un fond de teint fluide.

43. Procédé de maquillage de la peau, **caractérisé en ce qu'**il comprend l'application sur la peau d'au moins une composition selon l'une quelconque des revendications précédentes.

## Claims

1. Fluid cosmetic composition in the form of a water-in-oil emulsion comprising a liquid fatty phase, an aqueous phase and a dimethicone copolyol, **characterized in that** it comprises solid spherical particles of polymethyl methacrylate and **in that** the liquid fatty phase comprises isododecane, the said composition being free of cyclotetrasiloxane.

2. Fluid cosmetic composition in the form of a water-in-oil emulsion comprising a liquid fatty phase, an aqueous phase, a dimethicone copolyol and a C₈-C₂₂ alkyl dimethicone copolyol, **characterized in that** it comprises solid spherical particles of polymethyl methacrylate and **in that** the liquid fatty phase comprises a volatile hydrocarbon-based oil, the said composition being free of cyclotetrasiloxane.

3. Composition according to either of Claims 1 or 2, **characterized in that** the solid polymethyl methacrylate particles have a density ranging from 0.3 to 0.95, especially from 0.45 to 0.80 and more particularly from 0.5 to 0.75.

4. Composition according to either of claims 1 or 2, **characterized in that** it comprises at least two polymethyl methacrylates that are different in terms of density.

5. Fluid cosmetic composition in the form of a water-in-oil emulsion comprising a liquid fatty phase, an aqueous phase and a dimethicone copolyol, **characterized in that** it comprises solid particles of polymethyl methacrylate with at least two polymethyl methacrylates that are different in terms of density and **in that** the liquid fatty phase comprises isododecane, the said composition being free of cyclotetrasiloxane.

6. Composition according to either of claims 4 or 5, **characterized in that** it combines two types of polymethyl methacrylate particles whose difference in density is at least 0.12, especially at least 0.15, in particular at least 0.18 and more particularly at least 0.22.

7. Composition according to any one of Claims 4 to 6, **characterized in that** the polymethyl methacrylate particles are present in weight proportions ranging, respectively, from 40% to 60% by weight, especially from 45% to 55% and in particular from 48% to 52% relative to the total weight of polymethyl methacrylates.

8. Composition according to Claim 7, **characterized in that** the content of polymethyl methacrylate of higher density is predominant over the content of polymethyl methacrylate of lower density.

9. Composition according to any one of the preceding claims, **characterized in that** it comprises from 1% to 10% by weight, especially from 2% to 7% by weight and more particularly from 2.5% to 5.5% by weight of polymethyl methacrylate relative to the total weight of the the said composition.

10. Composition according to any one of Claims 1 to 9, **characterized in that** the dimethicone copolyol is a compound of formula (I) below: 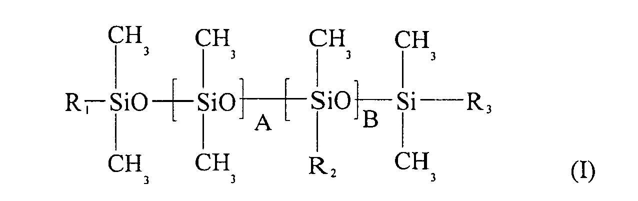 in which:
- R₁, R₂ and R₃, independently of each other, represent a C₁-C₆ alkyl radical or a radical -(CH₂)ₓ- (OCH₂CH₂)_{y}- (OCH₂CH₂CH₂)_{z}- OR₄, at least one radical R₁, R₂ or R₃ not being an alkyl radical, R₄ being a hydrogen, a C₁-C₃ alkyl radical or a C₂-C₄ acyl radical;
- A is an integer ranging from 0 to 200;
- B is an integer ranging from 0 to 50; with the condition that A and B are not simultaneously equal to 0;
- x is an integer ranging from 1 to 6;
- y is an integer ranging from 1 to 30; and
- z is an integer ranging from 0 to 5.

11. Composition according to Claim 10, **characterized in that** R₁ = R₃ = methyl radical, x is an integer ranging from 2 to 6 and y is an integer ranging from 4 to 30.

12. Composition according to Claim 10 or 11, **characterized in that** R₄ is a hydrogen atom.

13. Composition according to any one of Claims 10 to 12, **characterized in that** the dimethicone copolyol is a compound of formula (II) below: 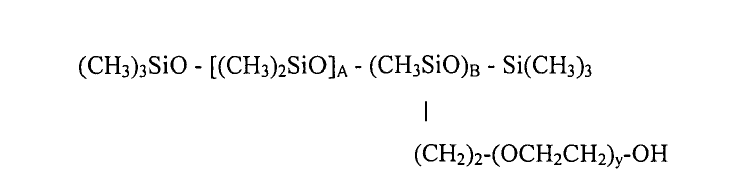 in which A is an integer ranging from 20 to 105, B is an integer ranging from 2 to 10 and y is an integer ranging from 10 to 20.

14. Composition according to Claim 10, **characterized in that** the dimethicone copolyol is a compound of formula (III) below:
HO - (CH₂CH₂O)_{y}- (CH₂)₃- [(CH₃)₂SiO]_{A'}- [(CH₃)₂Si]- (CH₂)₃- (OCH₂CH₂)_{y}- OH (III)
in which A' and y are integers ranging from 10 to 20.

15. Composition according to any one of Claims 10 to 14, **characterized in that** the dimethicone copolyol is present in a content ranging from 5% to 10% by weight, preferably ranging from 5% to 8% by weight and preferentially ranging from 5% to 7% by weight, relative to the total weight of the composition.

16. Composition according to any one of Claims 2 and 4 to 15, **characterized in that** the C₈-C₂₂ alkyl dimethicone copolyol is a compound of formula (IV) below: 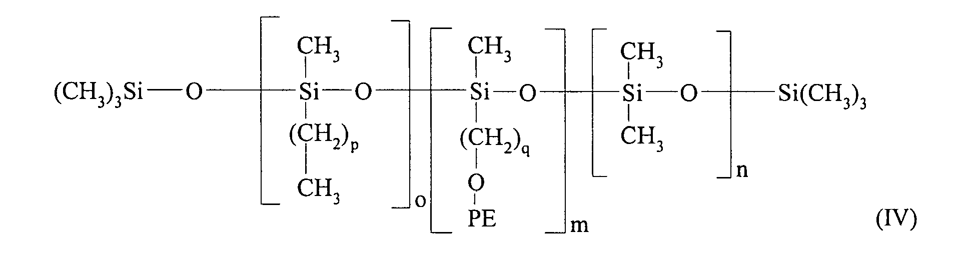 in which:
- PE represents (-C₂H₄O)ₓ-(C₃H₆O)_{y}-R, R being chosen from a hydrogen atom and an alkyl radical containing from 1 to 4 carbon atoms, x ranging from 0 to 100 and y ranging from 0 to 80, x and y not simultaneously being 0;
- m ranging from 1 to 40;
- n ranging from 10 to 200;
- o ranging from 1 to 100;
- p ranging from 7 to 21; and
- q ranging from 0 to 4.

17. Composition according to Claim 16, **characterized in that** R = H; m = 1 to 10; n = 10 to 100; o = 1 to 30; p = 15 and q = 3.

18. Composition according to any one of Claims 2 and 4 to 17, **characterized in that** the C₈-C₂₂ alkyl dimethicone copolyol is cetyl dimethicone copolyol.

19. Composition according to any one of Claims 2 and 4 to 18, **characterized in that** the C₈-C₂₂ alkyl dimethicone copolyol is present in a content ranging from 0.5% to 2% by weight and in particular ranging from 0.5% to 1.5% by weight relative to the total weight of the composition.

20. Composition according to any one of Claims 2 and 4 to 19, **characterized in that** the hydrocarbon-based volatile oil is chosen from linear or branched hydrocarbon-based volatile oils containing from 8 to 16 carbon atoms.

21. Composition according to Claim 20, **characterized in that** the volatile hydrocarbon-based oil is chosen from isododecane, isohexadecane and isohexyl neopentanoate, and mixtures thereof.

22. Composition according to any one of the preceding claims, **characterized in that** the volatile hydrocarbon-based oil, and especially isododecane, represents from 5% to 25%, especially from 10% to 20% and in particular from 10% to 15% by weight relative to the total weight of the composition.

23. Composition according to any one of the preceding claims, **characterized in that** the isododecane or the volatile hydrocarbon-based oil is combined with at least one distinct additional volatile oil chosen from hydrocarbon-based oils, fluoro oils and/or silicone oils optionally comprising alkyl or alkoxy groups, which are pendent or at the end of a silicone chain.

24. Composition according to Claim 23 **characterized in that** the silicone oil is a linear, branched or cyclic silicone oil with a viscosity at room temperature of less than 8 mm²/s and containing from 2 to 7 silicon atoms.

25. Composition according to Claim 23 or 24, **characterized in that** the silicone oil is chosen from octamethylcyclotetrasiloxane, decamethylcyclopentasiloxane, dodecamethylcyclohexasiloxane, heptamethylhexyltrisiloxane, heptamethyloctyltrisiloxane, hexamethyldisiloxane, octamethyltrisiloxane, decamethyltetrasiloxane and dodecamethylpentasiloxane, and mixtures thereof.

26. Composition according to any one of Claims 23 to 25, **characterized in that** the additional volatile oil represents from 20% to 32%, especially from 20% to 30% and more particularly from 22% to 26% by weight relative to the total weight of the composition.

27. Composition according to any one of the preceding claims, **characterized in that** the composition also comprises at least one non-volatile oil.

28. Composition according to Claim 27, **characterized in that** the non-volatile oil is chosen from hydrocarbon-based oils of mineral or synthetic origin, oils of animal origin, hydrocarbon-based oils of plant origin with a high triglyceride content consisting of fatty acid esters of glycerol, fatty acid esters, in particular containing from 4 to 22 carbon atoms, synthetic esters of formula R₁COOR₂ in which R₁ represents a linear or branched higher fatty acid residue containing from 7 to 40 carbon atoms and R₂ represents a branched hydrocarbon-based chain containing from 3 to 40 carbon atoms, hydroxylated esters, esters of aromatic acids and of alcohol containing from 4 to 22 carbon atoms, C₈ to C₂₆ higher fatty acids, C₈ to C₂₆ higher fatty alcohols, synthetic ethers containing at least 7 carbon atoms, silicone oils, polysiloxanes modified with fatty acids, fatty alcohols or polyoxyalkylenes, liquid fluorosilicones, and caprylic/capric acid triglycerides, and mixtures thereof.

29. Composition according to Claim 27 or 28, **characterized in that** it comprises 0.1% to 12% and especially 1% to 5% of at least one non-volatile oil, relative to the total weight of the composition.

30. Composition according to any one of the preceding claims, **characterized in that** it comprises at least one wax, at least one gum and/or at least one pasty fatty substance, which is silicone-based or non-silicone-based, of plant, animal, mineral or synthetic origin.

31. Composition according to any one of the preceding claims, **characterized in that** it comprises 0.01% to 10% by weight and especially 0.1% to 5% by weight of at least one wax relative to the total weight of the composition.

32. Composition according to any one of the preceding claims, **characterized in that** it comprises a thickener for the liquid fatty phase.

33. Composition according to Claim 32, **characterized in that** the thickener is chosen from organomodified clays and hydrophobic fumed silica.

34. Composition according to Claim 32 or 33, **characterized in that** the fatty-phase thickener is present in a content ranging from 0.1% to 5% by weight and especially from 0.4% to 3% by weight relative to the total weight of the composition.

35. Composition according to any one of the preceding claims, **characterized in that** the aqueous phase comprises at least one water-miscible organic solvent and/or stabilizers and/or at least one water-soluble or water-dispersible compound.

36. Composition according to any one of the preceding claims, **characterized in that** the said aqueous phase is present in a proportion of from 30% to 50% by weight and especially from 35% to 45% by weight relative to the total weight of the composition.

37. Composition according to any one of the preceding claims, **characterized in that** it also comprises at least one additional filler different from the polymethyl methacrylate powders.

38. Composition according to the preceding claim, **characterized in that** this filler is present in a proportion of from 0.1 % to 10% by weight and in particular from 0.1 % to 7% by weight relative to the total weight of the composition.

39. Composition according to any one of the preceding claims, **characterized in that** it also comprises a dyestuff chosen from lipophilic dyes, hydrophilic dyes, pigments and nacres.

40. Composition according to Claim 39, **characterized in that** the pigments are hydrophobic-coated pigments.

41. Composition according to Claim 39 or 40, **characterized in that** this dyestuff is present in a proportion of from 0.01% to 40% by weight, especially from 1% to 35% by weight, in particular from 2% to 25% by weight and more particularly from 3% to 20% by weight relative to the total weight of the composition.

42. Composition according to any one of the preceding claims, **characterized in that** it is in the form of a skin makeup composition and in particular a fluid foundation.

43. Process for making up the skin, **characterized in that** it comprises the application to the skin of at least one composition according to any one of the preceding claims.

## Patentansprüche

1. Fluide kosmetische Zusammensetzung in Form einer Wasser-in-Öl-Emulsion, umfassend eine flüssige Fettphase, eine wässrige Phase und ein Dimethicon-Copolyol, **dadurch gekennzeichnet, dass** sie kugelige feste Polymethylmethacrylat-Teilchen umfasst und dass die flüssige Fettphase Isododecan umfasst, wobei die Zusammensetzung frei von Cyclotetrasiloxan ist.

2. Fluide kosmetische Zusammensetzung in Form einer Wasser-in-Öl-Emulsion, umfassend eine flüssige Fettphase, eine wässrige Phase, ein Dimethicon-Copolyol und ein C₈-C₂₂-Alkyl-Dimethicon-Copolyol, **dadurch gekennzeichnet, dass** sie feste kugelige Polymethylmethacrylat-Teilchen umfasst und dass die flüssige Fettphase ein flüchtiges Kohlenwasserstofföl umfasst, wobei die Zusammensetzung frei von Cyclotetrasiloxan ist.

3. Zusammensetzung nach einem der Ansprüche 1 oder 2, **dadurch gekennzeichnet, dass** die festen Polymethylmethacrylat-Teilchen eine Dichte von 0,3 bis 0,9, insbesondere von 0,45 bis 0,80 und ganz besonders von 0,5 bis 0,75 besitzen.

4. Zusammensetzung nach einem der Ansprüche 1 oder 2, **dadurch gekennzeichnet, dass** sie mindestens zwei hinsichtlich der Dichte verschiedene Polymethylmethacrylate umfasst.

5. Fluide kosmetische Zusammensetzung in Form einer Wasser-in-Öl-Emulsion, umfassend eine flüssige Fettphase, eine wässrige Phase und ein Dimethicon-Copolyol, **dadurch gekennzeichnet, dass** sie feste Polymethylmethacrylat-Teilchen mit mindestens zwei hinsichtlich der Dichte verschiedenen Polymethylmethacrylaten umfasst und **dadurch**, dass die flüssige Fettphase Isododecan umfasst, wobei die Zusammensetzung frei von Cyclotetrasiloxan ist.

6. Zusammensetzung nach dem vorhergehenden Anspruch 4 oder 5, **dadurch gekennzeichnet, dass** sie zwei Typen von Polymethylmethacrylat-Teilchen kombiniert, deren Dichteunterschied mindestens 0,12, insbesondere mindestens 0,15, insbesondere mindestens 0,18 und ganz besonders mindestens 0,22 beträgt.

7. Zusammensetzung nach einem der Ansprüche 4 bis 6, **dadurch gekennzeichnet, dass** die hinsichtlich der Dichte verschiedenen Polymethylmethacrylat-Teilchen in Gewichtsanteilen von 40 bis 60 Gew.-%, insbesondere von 45 bis 55 Gew.-% bzw. insbesondere von 48 bis 52 Gew.-% bezüglich des Gesamtgewichts von Polymethylmethacrylaten variieren.

8. Zusammensetzung nach Anspruch 7, **dadurch gekennzeichnet, dass** der Gehalt an Polymethylmethacrylat mit höherer Dichte bezüglich des Gehalts an Polymethylmethacrylat mit geringerer Dichte höher ist.

9. Zusammensetzung nach einem der vorhergehenden Ansprüche, **dadurch gekennzeichnet, dass** sie 1 bis 10 Gew.-%, insbesondere 2 bis 7 Gew.-% und ganz besonders 2,5 bis 5,5 Gew.-% Polymethylmethacrylat bezüglich des Gesamtgewichts der Zusammensetzung enthält.

10. Zusammensetzung nach einem der Ansprüche 1 bis 9, **dadurch gekennzeichnet, dass** das Dimethicon-Copolyol eine Verbindung der folgenden Formel (I) ist: 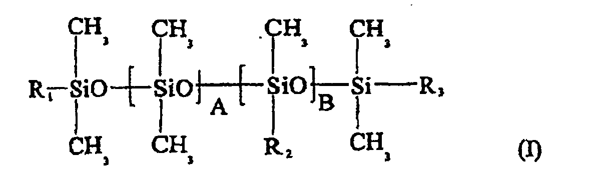 wobei:
- R₁, R₂, R₃ unabhängig voneinander einen C₁-C₆-Alkylrest oder einen Rest - (CH₂)ₓ-(OCH₂CH₂)_{y}-(OCH₂CH₂CH₂)ₓ-OR₄ darstellen, wobei mindestens ein Rest R₁, R₂ oder R₃ kein Alkylrest ist und R₄ ein Wasserstoff, ein C₁₋₃-Alkylrest oder ein C₂-C₄-Acylrest ist;
- A eine ganze Zahl von 0 bis 200 ist;
- B eine ganze Zahl von 0 bis 50 ist; mit der Maßgabe, dass A und B nicht gleichzeitig 0 sind;
- x eine ganze Zahl von 1 bis 6 ist;
- y eine ganze Zahl von 1 bis 30 ist; und
- z eine ganze Zahl von 0 bis 5 ist.

11. Zusammensetzung nach Anspruch 10, **dadurch gekennzeichnet, dass** R₁=R₃=Methylrest gilt, x eine ganze Zahl von 2 bis 6 ist und y eine ganze Zahl von 4 bis 30 ist.

12. Zusammensetzung nach Anspruch 10 oder 11, **dadurch gekennzeichnet, dass** R₄ ein Wasserstoffatom ist.

13. Zusammensetzung nach einem der Ansprüche 10 bis 12, **dadurch gekennzeichnet, dass** das Dimethicon-Copolyol eine Zusammensetzung der folgenden Formel (II) ist: 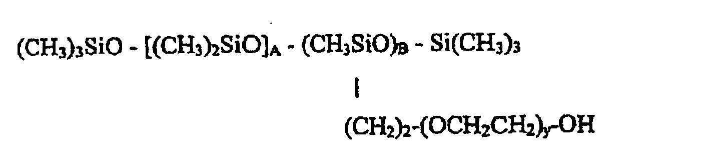 wobei A eine ganze Zahl von 20 bis 105 ist, B eine ganze Zahl von 2 bis 10 ist und y eine ganze Zahl von 10 bis 20 ist.

14. Zusammensetzung nach Anspruch 10, **dadurch gekennzeichnet, dass** das Dimethicon-Copolyol eine Verbindung der folgenden Formel (III) ist:
HO-(CH₂CH₂O)_{y}-(CH₂)₃-[(CH₃)₂SiO]_{A}-[(CH₃)₂Si]-(CH₂)₃-(OCH₂CH₂)_{y}-OH (III)
wobei A' und y ganze Zahlen von 10 bis 20 sind.

15. Zusammensetzung nach einem der Ansprüche 10 bis 14, **dadurch gekennzeichnet, dass** das Dimethicon-Copolyol in einem Gehalt von 5 bis 10 Gew.-%, insbesondere von 5 bis 8 Gew.-% und insbesondere von 5 bis 7 Gew.-% bezüglich des Gesamtgewichtes der Zusammensetzung vorhanden ist.

16. Zusammensetzung nach einem der Ansprüche 2 und 4 bis 15, **dadurch gekennzeichnet, dass** das C₈-C₂₂-Alkyl-Dimethicon-Copolyol eine Verbindung der folgenden Formel (IV) ist: 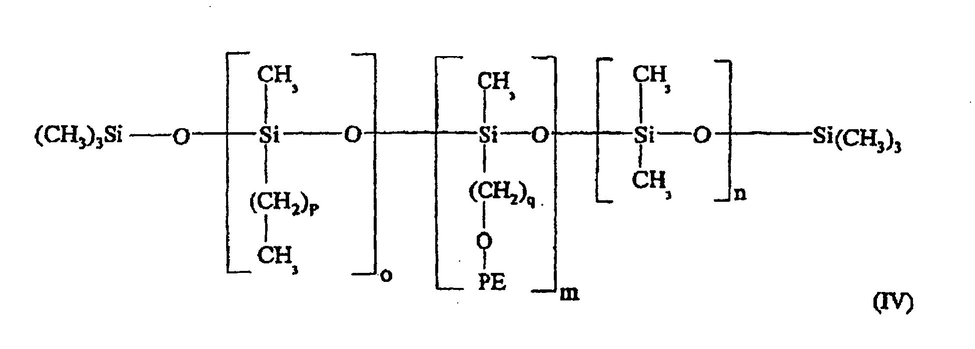 wobei
- PE (-C₂H₄O)ₓ-C₃H₆O)_{y}-R darstellt, R ausgewählt ist aus einem Wasserstoffatom und einem Alkylrest mit 1 bis 4 Kohlenstoffatomen, x 0 bis 100 ist und y 0 bis 80 ist, wobei x und y nicht gleichzeitig 0 sind;
- m 1 bis 40 ist;
- n 10 bis 200 ist;
- o 1 bis 100 ist;
- p 7 bis 21 ist; und
- q 0 bis 4 ist.

17. Zusammensetzung nach Anspruch 16, **dadurch gekennzeichnet, dass** R=H; m=1 bis 10; n=10 bis 100; 0=1 bis 30; p=15 und q=3 gilt.

18. Zusammensetzung nach einem der Ansprüche 2 und 4 bis 17, **dadurch gekennzeichnet, dass** das C₈-C₂₂-Alkyl-Dimethicon-Copolyol Cetyl-Dimethicon-Copolyol ist.

19. Zusammensetzung nach einem der Ansprüche 2 und 4 bis 18, **dadurch gekennzeichnet, dass** das C₈-C₂₂-Alkyl-Dimethicon-Copolyol in einem Gehalt von 0,5 bis 2 Gew.-% und insbesondere von 0,5 bis 1,5 Gew.-% bezüglich des Gesamtgewichts der Zusammensetzung vorhanden ist.

20. Zusammensetzung nach einem der Ansprüche 2 und 4 bis 19, **dadurch gekennzeichnet, dass** das flüchtige Kohlenwasserstofföl aus flüchtigen Kohlenwasserstoffölen mit 8 bis 16 linearen oder verzweigten Kohlenstoffatomen ausgewählt ist.

21. Zusammensetzung nach Anspruch 20, **dadurch gekennzeichnet, dass** das flüchtige Kohlenwasserstofföl aus Isododecan, Isohexadecan, Isohexylneopentanoat und ihren Gemischen ausgewählt ist.

22. Zusammensetzung nach einem der vorhergehenden Ansprüche, **dadurch gekennzeichnet, dass** das flüchtige Kohlenwasserstofföl und insbesondere Isododecan 5 bis 25, insbesondere 10 bis 20 und ganz besonders 10 bis 15 Gew.-% bezüglich des Gesamtgewichts der Zusammensetzung darstellt.

23. Zusammensetzung nach einem der vorhergehenden Ansprüche, **dadurch gekennzeichnet, dass** das Isododecan oder das flüchtige Kohlenwasserstofföl mit mindestens einem zusätzlichen unterschiedlichen flüchtigen Öl kombiniert ist, ausgewählt aus fluorierten und/oder silikonierten Kohlenwasserstoffölen, die gegebenenfalls Alkyl- oder Alkoxygruppe, die angehängt sind oder sich am silikonierten Kettenende befinden, umfassen.

24. Zusammensetzung nach Anspruch 23, **dadurch gekennzeichnet, dass** das silikonierte Öl ein lineares, verzweigtes oder cyclisches silikoniertes Öl mit einer Viskosität bei Umgebungstemperatur von unter 8 mm²/s mit 2 bis 7 Siliciumatomen ist.

25. Zusammensetzung nach Anspruch 23 oder 24, **dadurch gekennzeichnet, dass** das silikonierte Öl aus Octamethylcyclotetrasiloxan, Decamethylcyclopentasiloxan, Dodecamethylcyclohexasiloxan, Heptamethylhexyltrisiloxan, Heptamethyloctyltrisiloxan, Hexamethyldisiloxan, Octamethyltrisiloxan, Decamethyltetrasiloxan, Dodecamethylpentasiloxan und ihren Gemischen ausgewählt ist.

26. Zusammensetzung nach einem der Ansprüche 23 bis 25, **dadurch gekennzeichnet, dass** das zusätzliche flüchtige Öl 20 bis 32, insbesondere 20 bis 30 und ganz besonders 22 bis 26 Gew.-% bezüglich des Gesamtgewichts der Zusammensetzung darstellt.

27. Zusammensetzung nach einem der vorhergehenden Ansprüche, **dadurch gekennzeichnet, dass** die Zusammensetzung weiterhin mindestens ein nicht flüchtiges Öl umfasst.

28. Zusammensetzung nach Anspruch 27, **dadurch gekennzeichnet, dass** das nicht flüchtige Öl ausgewählt ist aus Kohlenwasserstoffölen, mineralischen oder synthetischen Ursprungs, Ölen tierischen Ursprungs, Kohlenwasserstoffölen pflanzlichen Ursprungs mit einem hohen Gehalt an Triglyceriden, bestehend aus Fettsäureestern und aus Glycerin, wobei die Fettsäureester insbesondere 4 bis 22 Kohlenstoffatome umfassen, synthetischen Estern der Formel R₁COOR₂, wobei R₁ den Rest einer höheren linearen oder verzweigten Fettsäure darstellt, die 7 bis 40 Kohlenstoffatome umfasst und R₂ eine verzweigte Kohlenwasserstoffkette darstellt, die 3 bis 40 Kohlenstoffatome enthält, hydroxylierten Estern, Estern von aromatischen Säuren und von Alkohol, die 4 bis 22 Kohlenstoffatomen umfassen, C₈-C₂₆-höheren Fettsäuren, C₈-C₂₆-höheren Fettalkoholen, synthetischen Ethern mit mindestens 7 Kohlenstoffatomen, silikonierten Ölen, mit Fettsäuren, Fettalkoholen oder Polyoxyalkylenen modifizierten Polysiloxanen, flüssigen fluorierten Silikonen, Triglyceriden von Caprylsäuren/Caprinsäure und ihren Gemischen.

29. Zusammensetzung nach Anspruch 27 oder 28, **dadurch gekennzeichnet, dass** sie 0,1 bis 12 % und insbesondere 1 bis 5 % bezüglich des Gesamtgewichts der Zusammensetzung von mindestens einem nicht flüchtigen Öl umfasst.

30. Zusammensetzung nach einem der vorhergehenden Ansprüche, **dadurch gekennzeichnet, dass** sie mindestens ein Wachs, mindestens ein Gummi und/oder mindestens einen pasteusen Fettkörper, der silikoniert ist oder nicht, pflanzlichen, tierischen, mineralischen oder synthetischen Ursprungs umfasst.

31. Zusammensetzung nach einem der vorhergehenden Ansprüche, **dadurch gekennzeichnet, dass** sie 0,01 bis 10 Gew.-% und insbesondere 0,1 bis 5 Gew.-% von mindestens einem Wachs bezüglich des Gesamtgewichts der Zusammensetzung umfasst.

32. Zusammensetzung nach einem der vorhergehenden Ansprüche, **dadurch gekennzeichnet, dass** sie ein Verdickungsmittel der flüssigen Fettphase umfasst.

33. Zusammensetzung nach Anspruch 32, **dadurch gekennzeichnet, dass** das Verdickungsmittel aus organisch modifizierten Tonmineralien und aus hydrophobem pyrogenem Siliciumdioxid ausgewählt ist.

34. Zusammensetzung nach Anspruch 32 oder 33, **dadurch gekennzeichnet, dass** das Verdickungsmittel der Fettphase in einem Gehalt von 0,1 bis 5 Gew.-% und insbesondere von 0,4 bis 3 Gew.-% bezüglich des Gesamtgewichts der Zusammensetzung vorhanden ist.

35. Zusammensetzung nach einem der vorhergehenden Ansprüche, **dadurch gekennzeichnet, dass** die wässrige Phase mindestens ein mit Wasser mischbares organisches Lösungsmittel und/oder Stabilisierungsmittel und/oder mindestens eine wasserlösliche oder wasserdispergierbare Verbindung umfasst.

36. Zusammensetzung nach einem der vorhergehenden Ansprüche, **dadurch gekennzeichnet, dass** die wässrige Phase in einem Verhältnis von 30 bis 50 Gew.-% und insbesondere von 35 bis 45 Gew.-% bezüglich des Gesamtgewichts der Zusammensetzung vorhanden ist.

37. Zusammensetzung nach einem der vorhergehenden Ansprüche, **dadurch gekennzeichnet, dass** sie weiterhin mindestens eine zusätzliche distinkte Charge von Polymethylmethacrylatpulvem umfasst.

38. Zusammensetzung nach dem vorhergehenden Anspruch, **dadurch gekennzeichnet, dass** diese Charge in einem Verhältnis von 0,1 bis 10 Gew.-% und insbesondere von 0,1 bis 7 Gew.-% bezüglich des Gesamtgewichts der Zusammensetzung vorhanden ist.

39. Zusammensetzung nach einem der vorhergehenden Ansprüche, **dadurch gekennzeichnet, dass** sie weiterhin ein Farbmittel, ausgewählt aus lipophilen Farbmitteln, hydrophilen Farbmitteln, Pigmenten und Pearl-Lacken, umfasst.

40. Zusammensetzung nach Anspruch 39, **dadurch gekennzeichnet, dass** die Pigmente hydrophobe beschichtete Pigmente sind.

41. Zusammensetzung nach Anspruch 39 oder 40, **dadurch gekennzeichnet, dass** diese färbende Substanz in einem Verhältnis von 0,01 bis 40 Gew.-%, insbesondere von 1 bis 35 Gew.-% und besonders von 2 bis 25 Gew.-% und ganz besonders von 3 bis 20 Gew.-% bezüglich des Gesamtgewichts der Zusammensetzung vorhanden ist.

42. Zusammensetzung nach einem der vorhergehenden Ansprüche, **dadurch gekennzeichnet, dass** sie in Form einer Schminkzusammensetzung für die Haut und insbesondere eines Fond de teint-Fluides vorliegt.

43. Schminkverfahren für die Haut, **dadurch gekennzeichnet, dass** es das Aufbringen auf die Haut von mindestens einer Zusammensetzung nach einem der vorhergehenden Ansprüche umfasst.
